(19) Europäisches Patentamt
European Patent Office
Office européen des brevets

(11) Publication number: **0 081 320 A2**

# (12) EUROPEAN PATENT APPLICATION

(21) Application number: 82306275.7

(22) Date of filing: 25.11.82

(51) Int. Cl.[3]: C 07 C 143/68
C 07 D 333/24, C 07 C 139/00

(30) Priority: 07.12.81 GB 8136825

(43) Date of publication of application: 15.06.83 Bulletin 83/24

(84) Designated Contracting States: BE CH DE FR GB IT LI NL SE

(71) Applicant: BEECHAM GROUP PLC
Beecham House Great West Road
Brentford Middlesex(GB)

(72) Inventor: Taylor, Andrew William
7 Doods Road
Reigate Surrey(GB)

(72) Inventor: Guest, Angela Wendy
Golding 66 Strathconar Avenue
Little Bookham Surrey(GB)

(72) Inventor: Lashford, Andrew Gerard
15 Lake Road North Roath Park
Cardiff Wales(GB)

(74) Representative: Hesketh, Alan, Dr. et al,
European Patent Attorney Beecham Pharmaceuticals
Great Burgh Yew Tree Bottom Road
Epsom, Surrey, KT18 5XQ(GB)

## (54) Preparation of acetic acid derivatives.

(57) A process for the preparation of a compound of formula (I) or a salt thereof:

$$R^1.CH(SO_3R^2).CO_2H \quad (I)$$

wherein $R^1$ is an optionally substituted aryl group and $R^2$ is hydrogen, a salting ion or a sulphonyl ester forming group; *characterised in that* a mono-deprotonated methylene compound of the formula (II):

$$R^1.\overset{\ominus}{C}H(SO_3R^2) \quad (II)$$

wherein $R^1$ and $R^2$ are as defined with respect to formula (I) and wherein any reactive groups may be protected, is reacted with carbon dioxide.

EP 0 081 320 A2

# Process for the Preparation of Acetic Acid Derivatives

This invention relates to a chemical process for the preparation of α-sulpho acetic acid derivatives and in particular to α-sulpho acetic acids wherein the α-sulpho group is esterified.

European Patent Publication Number 0015690 A1 discloses *inter alia* penicillin derivatives having a sulphonic acid or sulphonyl ester grouping in the side chain. One process disclosed for the preparation of the penicillin derivatives comprises reacting an ester of 6,β-amino-6,α-methoxy penicillanic acid with an activated derivative of an acid of formula (A):

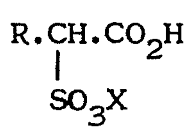

(A)

wherein R is $C_{1-6}$ alkyl; an optionally substituted 5-membered heterocyclic ring containing one or two heteroatoms selected from oxygen, sulphur and nitrogen; phenyl; mono-substituted phenyl where the substituent is

halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino (for example $-NHSO_2CH_3$); or di-substituted phenyl where the substituents are selected from hydroxy, halogen, methoxy, acetoxy and amino; and X is hydrogen or $C_{1-6}$ alkyl.

The present invention provides a process for the preparation of a compound of formula (I) or a salt thereof:

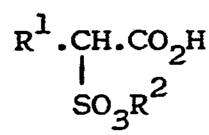

(I)

wherein $R^1$ is an optionally substituted aryl group and $R^2$ is hydrogen, a salting ion or a sulphonyl ester forming group;

<u>characterised in that</u> a mono-deprotonated methylene compound of the formula (II):

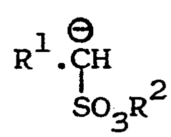

(II)

wherein $R^1$ and $R^2$ are as defined with respect to formula (I) and wherein any reactive groups may be protected, is reacted with carbon dioxide;

and thereafter optionally carrying out one or more of the following steps:

a) converting a salt to the corresponding free acid or to a different salt

and b) removing any protecting groups on $R^1$ and $R^2$.

Suitable aryl groups $R^1$ include an optionally substituted heteroaromatic ring containing one or two atoms selected from oxygen, sulphur and nitrogen, or an optionally substituted phenyl group.

Suitable substituents for the heteroaromatic and the phenyl group include $C_{1-6}$ alkyl, phenyl, halogen, $C_{1-6}$ alkoxy, amino, nitro, hydroxy, $C_{1-6}$ alkylamido, $C_{1-6}$ alkylcarbonyloxy, carboxy, $C_{1-6}$ alkoxycarbonyl, aralkyloxycarbonyl, halo $(C_{1-6})$ alkyl, $C_{1-6}$ alkylcarbonyl, aryloxy, arylcarbonyl, $C_{1-6}$ alkylsulphonyl $C_{1-6}$ alkylamino, di-$C_{1-6}$ alkylamino, cyano or thio. The amino, hydroxy, carboxy, $C_{1-6}$ alkylamino, and thio groups are examples of substituents, for the heteroaromatic and the phenyl group which are normally protected prior to the process of the present invention.

Suitable heteroaromatic ring systems include pyridyl, thienyl, furanyl and thiazole.

Preferably the group $R^1$ is phenyl, p-hydroxyphenyl, p-aminophenyl, 2-thienyl or 3-thienyl.

Suitable sulphonyl ester forming groups $R^2$ include those which may be converted to the free sulphonic acid subsequent to coupling the carboxylic acid (I) to a penicillin or cephalosporin nucleus.

Aptly $R^2$ is hydrogen or a $C_{1-6}$ alkyl group such as, for example, methyl, ethyl, _n_- or _iso_-propyl, and _n_-, _sec_-, _tert_- or _iso_-butyl.

Preferably $R^2$ is an ethyl or _iso_-butyl group.

The generation of the mono-deprotonated methylene compound (II) and the subsequent reaction with carbon dioxide are conveniently carried out without intermediate isolation of the compound (II).

Suitable precursors for the mono-deprotonated methylene compound (II) include a compound of formula (III):

$$R^1-CH_2-SO_3R^2 \qquad \text{(III)}$$

wherein $R^1$ and $R^2$ are as defined hereinbefore with respect to formula (I) and wherein any reactive groups may be protected.

The mono-deprotonated methylene compound (II) may be generated from the compound (III) by reaction with strong base.

Suitable bases for deprotonating compound (III) include alkyl Grignard reagents such as, for example, isopropylmagnesium bromide; organolithium reagents such as methyl lithium, butyl lithium, phenyl lithium, and lithium naphthalene; organosodium reagents such as sodium naphthalene and sodium phenanthrene; and saline hydrides such as sodium hydride.

The preferred base for deprotonating compound (III) is butyl lithium.

Suitable solvents in which the deprotonation reaction and subsequent reaction with carbon dioxide may be performed will be aprotic such as, for example, diethylether, tetrahydrofuran, dimethylformamide, hexamethylphosphoramide and quinoline. The reactions are generally carried out at moderate to low temperatures _ie_ in the range -100°C to +30°C. The course of the reaction may be followed by conventional methods such as thin layer chromatography and terminated when an optimum quantity of product is present in the reaction mixture.

The mono-deprotonated methylene compound (II) and the strong base base used in its generation are reactive materials and to ensure reasonable yields all reagents, solvents, apparatus and the carbon dioxide should be

dried accordingly and the reactions are preferably performed in vacuo or under an inert atmosphere. Suitable apparatus and methods for performing the reactions are disclosed in D.F. Schriver, "The Manipulation of Air Sensitive Materials" McGraw Hill Book Company, New York 1969.

The starting material of formula (III) may be prepared by known literature methods, when $R^1$ is phenyl. For example, compound (III) may be prepared by a process such as that disclosed in DE-OS 2502912.

A particularly useful method for preparing the starting material (III) wherein $R^1$ is thienyl comprises the reaction of a thienylmethylhalide with a sulphite salt. Suitable thienylmethylhalides include 3-thienylmethylbromide and 2-thienylmethylchloride. A suitable sulphite salt for use in the above process is sodium sulphite. The thienylmethyl sulphonic acid salt obtained in the above process may be used directly in the process of the present invention, or may be esterified or converted to the free acid before use. A particularly suitable method for synthesising 3-thienylmethylsulphonic esters comprises reaction of the sodium salt of 3-thienylmethylsulphonic acid with an organic halide in the presence of silver tetrafluoroborate; this method is particularly suitable for producing the $C_{1-6}$ alkyl esters such as, for example the iso-butyl ester. A preferred halide for use in this process is the bromide. A particularly suitable method for synthesising 2-thienylmethylsulphonic $C_{1-6}$ alkyl esters comprises reaction of a tetraalkylammonium salt of 2-thienylmethyl sulphonic acid with a trialkyloxonium salt; this method is particularily suitable for the ethyl ester of 2 thienyl methyl sulphonic acid.

The compounds of formula III wherein $R^1$ is thienyl are novel intermediates and as such constitute a further aspect of this invention.

The compounds of formula (I) produced by the process of the present invention have utility as intermediates in the preparation of penicillins and penicillin derivatives such as, for example, those described in British Patents Numbers 1530861 and 1277578, and European Patent Application Number 0015690 A1. The compounds of formula (I) produced by the process of the present invention are particularly useful as intermediates in the synthesis of a compound of formula (IV) or a pharmaceutically acceptable salt or <u>in vivo</u> hydrolysable ester thereof:

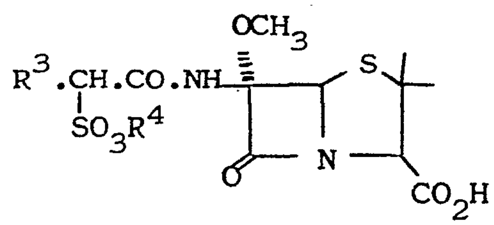

(IV)

wherein $R^3$ is an optionally substituted 5-membered heterocyclic aromatic ring containing one or two heteroatoms selected from oxygen, sulphur and nitrogen; phenyl; mono-substituted phenyl where the substituent is halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino (for example $-NHSO_2CH_3$); or di-substituted phenyl where the substituents are selected from hydroxy, halogen, methoxy, acetoxy and amino; and $R^4$ is hydrogen or $C_{1-6}$ alkyl.

One subgroup of penicillin derivatives which may be prepared using a compound of formula (I) produced by the process of the present invention as an intermediate is the compounds of formula (V) or a pharmaceutically acceptable salt or <u>in vivo</u> hydrolysable ester thereof:

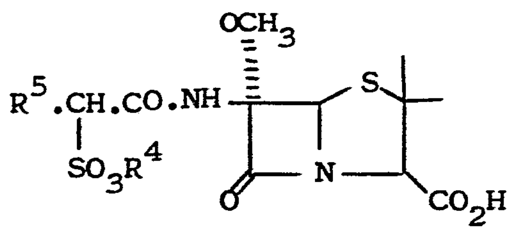

(V)

wherein $R^4$ is as defined hereinbefore with reference to formula (IV) and $R^5$ is 2-thienyl, 3-thienyl, phenyl, mono-substituted phenyl where the substitutent is halogen, $C_{1-6}$ alkoxy, amino or $C_{1-6}$ alkyl, or disubstituted phenyl where the substituents are selected from hydroxy, halogen, methoxy, acetoxy and amino.

The following examples illustrate the process of the present invention.

Example 1 2-(2-Methylpropylsulphonyl)phenylacetic acid

a) 2-Methylpropyl benzylsulphonate

Benzylsulphonyl chloride (1.9 g, 10 mmole) was dissolved in 2-methylpropanol (15 ml) and pyridine (1 ml) and refluxed for ½ hour. The solvent was removed under reduced pressure and the residue dissolved in chloroform (50 ml). This was washed with saturated sodium bicarbonate solution, dilute hydrochloric acid and saturated brine, dried and evaporated to a give a low melting solid (0.98 g, 43%); mp 30-31$^{o}$, ν max (Nujol) 1170, 980, 950, 780, 700 $cm^{-1}$; δ ($CDCl_3$) 0.94 (6H, d, J 7.5 Hz, 2 x $CH_3$), 1.92 (1H, m, CH), 3.85 (2H, d, J 6.5 Hz, $SO_2CH_2$), 4.35 (2H, S, $\underline{CH_2}$Ph), 7.43 (5H, m, Ph).

b) 2-(2-Methylpropylsulphonyl)phenylacetic acid

2-Methylpropyl benzylsulphonate (4.44 g) in dry tetrahydrofuran (35 ml) was added dropwise to a solution of 2M n-butyl lithium (12 ml) at -78°. The orange solution was stirred at -70° for 30 minutes. This was then poured onto solid carbon dioxide and allowed to come to room temperature. The solvent was removed under reduced pressure and the residue dissolved in ethyl acetate (50 ml). This was then extracted with dilute sodium bicarbonate (3 x 50 ml) and the combined extracts acidified to pH 2.5. This was then extracted with ethyl acetate (3x 50 ml). The combined extracts were dried over magnesium sulphate and evaporated to an oil. (3.33 g, 63%) δ ($CDCl_3$) 0.87 (6H, d, J 7.5 Hz, 2 x $CH_3$), 2.00 (1H, m, $CH_2$<u>CH</u>$(CH_3)_2$), 3.95 (2H, d, J 6.5 Hz, <u>CH</u>$_2CH(CH_3)_2$), 5.34 (1H, s, Ph<u>CH</u>), 7.25 - 7.90 (5H, m, Ph), 10.36 (1H, broad, $CO_2H$).

## Example 2 2-Isobutylsulphonyl thien-3'-yl acetic acid

### a) Sodium 3-thenyl sulphonate

3-Thenylbromide (68.32 g, 0.37 mol) in ethanol (40 ml) was added to sodium sulphite heptahydrate (93 g, 0.37 mol) in water (200 ml) and stirred at 60$^o$ for 4.5 hours. The reaction mixture was evaporated to dryness and the residue treated with hot ethanol to leave the title compound in quantitative yield. δ ($D_2O$) 4.27 (2H, s, $CH_2$), 7.33 (1H, m, thiophene protons), 7.52 (2H, m, thiophene protons), ν max (Nujol) 1145, 1190, 1230 $cm^{-1}$.

### b) Isobutyl 3-thenyl sulphonate (Method 1)

Sodium 3-thenyl sulphonate (1.06 g, 3.3 mmol) in DMF (30 ml) was treated with silver tetrafluoroborate (1.3 g, 6.6 mmol) and isobutylbromide (3.6 ml, 33 mmol) and stirred at 65$^o$ for 18 hrs. The reaction was allowed to cool, diluted with ethyl acetate and filtered. The filtrate was washed with water, brine, dried and evaporated to give the title compound (129 mg, 17%). δ ($(CD_3)_2CO$) 0.92 (6H, d, J, 6Hz, $CH_3$), 1.97 (1H, nonet, J 6Hz, CH), 3.98 (2H, d, J 6Hz, $SO_3CH_2$), 4.65 (2H, s, $CH_2$), 7.35 (1H, m, thiophene protons), 7.63 (2H, m, thiophene protons). ν max ($CH_2Cl_2$) 1170, 1350, 1675 $cm^{-1}$.

c) Isobutyl 3-thenyl sulphonate (Method 2)

3-Thenyl sulphonic acid sodium salt (0.64 g, 2.0 mmol) was added to thionyl chloride (4 ml) containing DMF (0.016 ml, 0.2 mmol), and stirred overnight at room temperature. Diisopropyl ether (20 ml) was added, the mixture filtered, and the filtrate concentrated to ca. 2 ml. This was again diluted and reconcentrated as before, to give a solution containing 3-thenyl sulphonyl chloride, ν max ($CH_2Cl_2$) 1380, 1175 $cm^{-1}$. Isobutanol (10 ml), triethylamine (0.28 ml, 2.0 mmol) and 4-dimethylaminopyridine (24 mg) were sequentially added, and the solution stirred for 2 hours at room temperature. Ether was added, and the organic solution washed with dilute hydrochloric acid, sodium bicarbonate solution and brine, dried ($Na_2SO_4$) and evaporated to give the title compound, chromatographed on silica gel (20:1 petrol: ethyl acetate), affording 0.26 g (59%), spectral details as above.

d) 2-Isobutylsulphonyl thien-3'-yl acetic acid

Isubutyl 3-thenyl sulphonate (0.23 g, 1.0 mmol) in T.H.F. (10 ml) at -70$^{o}$ was treated dropwise under $N_2$ with 2N BuLi solution in hexane (0.55 ml, 1.1 mmol), and the solution stirred 30 mins at -70$^{o}$. A slurry of solid carbon dioxide in T.H.F. (50 ml) was prepared and stirred. The reaction solution above was added to the carbon dioxide slurry; a drying tube was fitted and the mixture allowed to warm to room temperature. The solution was concentrated, diluted with ethyl acetate (50 ml), and the organic layer extracted with water (20 ml) and sodium bicarbonate solution (20 ml). The aqueous extracts, combined, were acidified to pH 2 and extracted

with ethyl acetate (2 x 50 ml). The organic extracts were washed with brine, dried ($Na_2SO_4$) and evaporated to give the title compound (0.25 g, 90%), ν max ($CH_2Cl_2$) 1710, 1360, 1170 $cm^{-1}$, δ ppm [$(CD_3)_2CO$] 0.89 (6H, d, J 6Hz, $CH_3$), 1.7-2.4 (1H, m, $-CHMe_2$) 4.01 (2H, d, J 6Hz, $-SO_3CH_2$), 5.75 (1H, s, $-CHSO_3$-), 7.48 (2H, m, thiophene protons), 7.83 (1H, m, thiophene proton), 8.81 (1H, s, -OH).

Example 3 2-Ethylsulphonyl thien-2'-yl acetic acid

a) Sodium 2-thenyl sulphonate

2-Thenyl chloride (6.04 g, 45.6 mmol) in methanol (20 ml) was added to sodium sulphite (11.7 g, 46 mmol) in water (30 ml), and the solution stirred at 60$^{o}$ for five hours, cooled, and evaporated. The residue was extracted with boiling ethanol (0.75 l) and the resultant solution concentrated. Crystallisation gave the title compound (2.31 g; 20%), δppm ($D_2O$) 7.39 (1H, m, thiophene proton), 7.12 (2H, m, thiophene protons), 4.40 (2H, s, $CH_2$).

b) Ethyl 2-thenyl sulphonate

Sodium 2-thenyl sulphonate (0.14 g, 0.5 mmol) and cetyl trimethylammonium bromide (0.18 g., 0.5 mmol) were shaken with methylene chloride: water. The organic layer was evaporated to give cetyl trimethylammonium 2-thenyl sulphonate, used without purification. This was dissolved in methylene chloride and treated with triethyloxonium tetrafluoroborate (96 mg., 0.5 mmol) in methylene chloride (0.4 ml). After 90 mins stirring at room temperature, the solution was diluted with methylene chloride (20 ml), washed with water (2 x 10 ml), dried ($Na_2SO_4$) and evaporated. The desired product was purified by passing through fluorosil (10 g) in ethyl acetate, to give the title compound (0.05 g., 50%), ν max ($CH_2Cl_2$), 1360, 1175 $cm^{-1}$, δppm ($CDCl_3$) 1.36 (3H, t, J 7Hz, $CH_3C$-O), 4.25 (2H, q, J 7Hz, $-CH_2$-O), 4.61 (2H, s, $-CH_2-SO_3-$), 7.25 (3H, m, thienyl protons).

g) 2-Ethylsulphonyl thien-2'-yl acetic acid

Ethyl 2-thenyl sulphonate (0.34 g., 1.65 mmol) in T.H.F. (20 ml) at -70$^{o}$ was treated with n-butyl lithium (0.85 ml, 1.7 mmol) in hexane (2N). After 30 minutes, the solution was added to a stirred suspension of solid carbon dioxide in T.H.F. (100 ml), and the mixture allowed to warm to room temperature. The solution was concentrated, diluted with ethyl acetate (50 ml) and extracted with water (20 ml) and sodium bicarbonate solution (20 ml). The combined aqueous extracts were acidified to pH2, and extracted with ethyl acetate (2 x 50 ml). The organic extracts were brine washed (20 ml), dried ($Na_2SO_4$) and evaporated to give the title product (0.20 g., 48%). ν max ($CH_2Cl_2$) 1720, 1360, 1180 $cm^{-1}$, δppm [$(CD_3)_2CO$] 1.29, (3H, t, J 7Hz, $-O-C-CH_3$), 4.34 (2H, q, J 7Hz, $-O-CH_2-$), 5.94 (1H, s, $-CH-SO_3-$), 7.36 (3H, complex, thienyl protons).

Claims

1. A process for the preparation of a compound of formula (I) of a salt thereof:

$$\begin{array}{l} R^1.CH.CO_2H \\ \quad\;\; | \\ \quad\;\; SO_3R^2 \end{array} \qquad (I)$$

wherein $R^1$ is an optionally substituted aryl group and $R^2$ is hydrogen, a salting ion or a sulphonyl ester forming group;

characterised in that a mono-deprotonated methylene compound of the formula (II):

$$\begin{array}{l} R^1.\overset{\ominus}{C}H \\ \quad\;\; | \\ \quad\;\; SO_3R^2 \end{array} \qquad (II)$$

wherein $R^1$ and $R^2$ are as defined with respect to formula (I) and wherein any reactive groups may be protected, is reacted with carbon dioxide;
and thereafter optionally carrying out one or more of the following steps:

a) converting a salt to the corresponding free acid or to a different salt and

b) removing any protecting groups on $R^1$ and $R^2$.

2. A process as claimed in claim 1 wherein $R^1$ is an optionally substituted heteroaromatic ring containing one or two atoms selected from oxygen, sulphur and nitrogen, or an optionally substituted phenyl group.

3. A process as claimed in claim 2 wherein the heteroaromatic and the phenyl group are substituted by $C_{1-6}$ alkyl, phenyl, halogen, $C_1$-6 alkoxy, amino, nitro hydroxy, $C_{1-6}$ alkylamido, $C_{1-6}$alkylcarbonyloxy, carboxy $C_{1-6}$alkylcarbonyl, aralkyloxycarbonyl, halo $(C_{1-6})$ alkyl, $C_{1-6}$alkylcarbonyl, arloxy, arylcarbonyl, $C_{1-6}$ alkylsulphonyl $C_{1-6}$alkylamino, di-$C_{1-6}$alkylamino, cyano or thio groups.

4. A process as claimed in any one of claims 1 to 3 wherein $R^1$ is pyridyl, thienyl, furanyl or thiazolyl.

5. A process as claimed in any one of claims 1 to 3 wherein $R^1$ is phenyl, p-hydroxyphenyl, p-aminophenyl, 2-thienyl or 3-thienyl.

6. A process as claimed in any one of claims 1 to 5 wherein $R^2$ is hydrogen or a $C_{1-6}$alkyl group.

7. A process as claimed in any one of claims 1 to 6 wherein R is ethyl or iso-butyl.

8. A process as claimed in claim 1 wherein the precursor for the mono-deprotonated methylene compound (II) is a compound of formula (III):

$$R^1-CH_2-SO_3R^2 \qquad (III)$$

wherein $R^1$ and $R^2$ are as defined hereinbefore with respect to formula (I) and wherein any reactive groups may be protected.

9. A process as claimed in claim 8 wherein the mono-deprtonated methylene compound (II) is generated from the compound (III) by reaction with strong base.

10. A process as claimed in claim 9 wherein the strong base is butyl lithium.